# EUROPEAN PATENT SPECIFICATION

(11) **EP 0 455 282 B1**
(45) Date of publication and mention of the grant of the patent: **28.12.1994**
(21) Application number: 91200746.5
(22) Date of filing: 29.03.1991
(51) Int. Cl.: A61B 17/02

(54) **Surgical retractor in particular for cholecystectomy**
Chirurgischer Retraktor, insbesondere zur Cholezystektomie
Ecarteur chirurgical, surtout destiné à cholécystectomie

(30) Priority: 03.04.1990 IT 1993390
(43) Date of publication of application: 06.11.1991
(73) Proprietor: Amato, Giuseppe, I-90144 Palermo (IT)
(72) Inventor: Amato, Giuseppe, I-90144 Palermo (IT)
(74) Representative: Fusina, Gerolamo

(56) References cited:
- DE-A- 618 652
- GB-A- 2 078 526
- US-A- 2 751 903
- US-A- 3 467 079

## Description

This invention relates to a surgical retractor, particularly for cholecystectomy.

In the current state of the art, a surgeon generally works with a series of instruments forming the normal equipment to an operating table. In most cases these instruments are multi-functional in the sense that they can serve the same purpose or similar purposes in numerous types of operation.

Because of their generalized structure these instruments require the surgeon to exercise greater commitment and ability to enable them to be used correctly in different situations. In this respect he has to be particularly attentive in order to overcome any deficiencies deriving from the generalized structure of the instruments.

In addition in the particular case of microsurgery these instruments cannot be used correctly in most cases.

In the case of cholecystectomies, the operation is performed by making an incision currently of about twenty centimetres. The edges of the cut are kept apart using instruments usually also used for other types of operation.

From US-A-3 467 079 a retractor for surgical operation on the gall bladder is known, which comprises a shaft and a blade removably secured thereto. The shaft terminates in a hook and is adjustably fixed to a retractor frame comprising two substantially parallel arms for keeping the operation area open. Such a retractor requires an incision the size of which is relatively large compared with the size of the effective operation area. This size is mainly due to the need to reach an organ (the gall-bladder) located deep within the patient's body and submerged by other viscera.

It is however well known that it is the size of an abdominal surgical incision which mostly determines the duration of the post-operative stage by externally exposing the abdominal viscera for a time such as to induce reflected intestinal paralysis, the duration of which is greater the more extensive and prolonged is the exposure of said viscera. Consequently reducing the incision and hence the operation time would have undoubted advantages both for the patient and for the logistic structure required for his assistance during the post-operative stage.

From GB-A-2 078 526 a surgical retractor provided with illumination means is known which allows to illuminate the effective operational area, so improving identifiability of surface structures even within deep operation wounds. However, also this known retractor does not solve satisfactorily the problems connected with the extension of the wound relative to the effective operation area.

US-A-2 751 903 discloses a surgical retractor according to the preamble of claim 1. The known retractor is easily and quickly adjustable both as to length and width, and also as to the outline shape of its spread condition. The retractor is provided with means by which an attained adjusted width is automatically retained by the tensional pressure exerted upon the retractor by spread margins of the incision in which the retractor is engaged.

However, also this known adjustable retractor involves an incision relatively large compared with the size of the effective operation area.

The object of the present invention is to provide a surgical retractor particularly for cholecystectomy, which overcomes the aforesaid drawbacks by enabling the access incision particularly to the gall-bladder to be kept open during the operation while at the same time localizing the organ in such a manner that the surgeon is able to operate comfortably by gaining deep access to it via an incision of dimensions which are basically small compared with the area of effective operation.

This object is attained by a surgical retractor having the features claimed in claim 1.

The invention is illustrated by way of non-limiting example in the Figures of the accompanying drawings.
Figure 1 is a perspective view of the retractor in the open position;
Figure 2 is a side elevational view of the retractor in the closed position;
Figure 3 is a plan view of the retractor in the closed position;
Figure 4 is an opposite plan view to the preceding, showing the retractor in the closed position;
Figure 5 is a front view of an alternative form of a dilator.

With reference to said figures, the surgical retractor of the invention, indicated overall by the reference numeral 1, is of the type usable particularly but not exclusively for cholecystectomy operations.

Said retractor 1 comprises a retractor frame 2, means 3 for locking said frame 2 in an autostatic position, and dilators 4.

The retractor frame 2 is formed by a series of sides 5 joined together by hinges to form an articulated polygonal frame. In the illustrated example the retractor frame 2 is an articulated quadrilateral, at the vertices of which there are two pairs of opposite hinges 6 respectively 6A comprising pins 8 and 8A respectively.

The hinges 6A have their axes of rotation 16A converging at an angle α indicatively between 50° and 60°, whereas the remaining hinges 6 have their axes 16 parallel, the reason for this being clarified hereinafter.

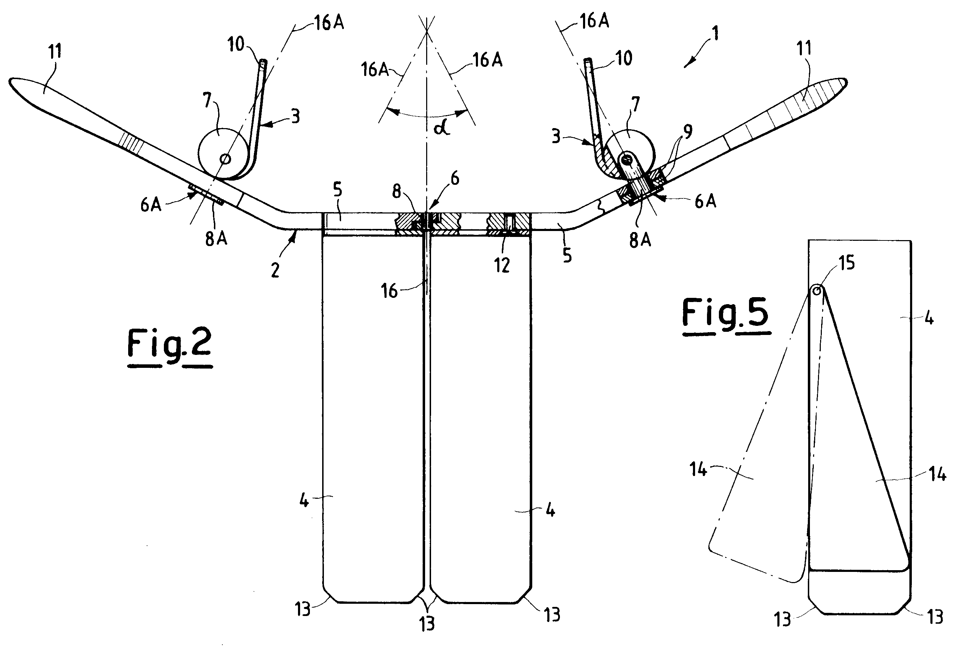

As visible in Fig. 2, the axes 16A converge at a point situated opposite to the dilators 4 relative to the retractor frame 2.

The means 3 for locking the frame in an autostatic position comprise in the illustrated example two cams 7 manually operable by respective levers 10 and acting via respective pins 8A on respective pairs of toothed jaws 9 rigid with two mutually hinged sides 5 of the retractor frame 2.

In the illustrated example the locking means are provided at the hinges 6A, and the toothed jaws 9 are formed directly on the sides 5 which converge at said hinges 6A.

At each hinge 6A the hinged sides 5 are prolonged to form a pair of arms 11 arranged such that when made to approach each other they automatically cause the retractor frame 2 to widen and hence open.

The position of maximum opening of the frame 2 is attained when the arms 11 of each pair are brought into touching contact. The dilators 4 are secured to the retractor frame 2, and in the illustrated example they are removably secured by screws 12 to the sides 5 of the retractor frame 2. In this manner one group of dilators 4 can be quickly and easily replaced by another. Alternatively, the dilators 4 can be fixed irremovably to the retractor frame 2.

The dilators 4 are substantially of thin plate form and extend along an axis which cuts the retractor frame 2. The dilators 4 have an indicative length of between 6 and 12 cm and are chosen on the basis of the size of the patient and/or the depth of the effective operating region within the patient's body.

The number of dilators 4 is usually equal to the number of sides 5 of the retractor frame 2. However if the retractor 1 is used for cholecystectomies the number of dilators 4 is at least one less than the number of sides 5. In the illustrated example the dilators 4 are three in number.

This is because of the particular position of the gall-bladder relative to the other viscera, in that the presence of a further dilator 4 beyond the three would obstruct access to the gall-bladder.

Instead of a missing dilator 4, one dilator could possibly be provided having a suitably chosen length different from that of the others.

The dilators 4 have a substantially arcuate cross-section and are arranged with their concavity facing the interior of the retractor 1. The dilators 4 have their outer ends 13 bevelled.

The purpose of the retractor 1 is to keep the incision open during the operation and at the same time separate the viscera which would otherwise interfere with the effective operating region.

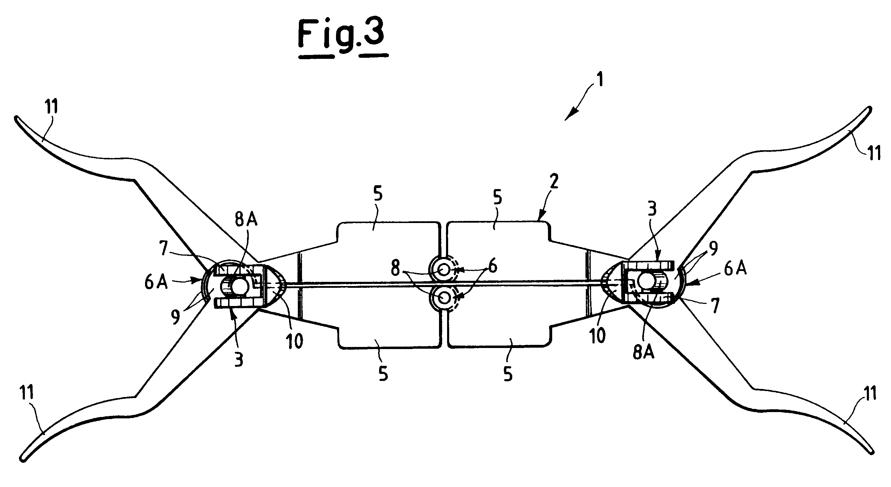

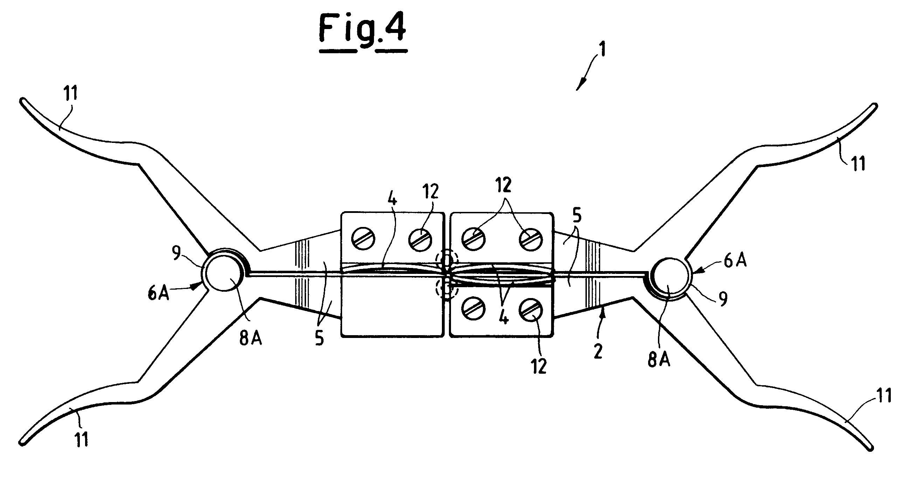

The retractor 1 is inserted into the incision in the closed position (see Figures 2, 3 and 4) and possibly locked in this position by the locking means 3.

This insertion is facilitated by the flat arrangement of the dilators 4 with their axis perpendicularly cutting the retractor frame 2, their platelike structure and the presence of the bevelled ends 13. The length of the dilators 4 enables effective operating areas deep within the patient's body to be reached.

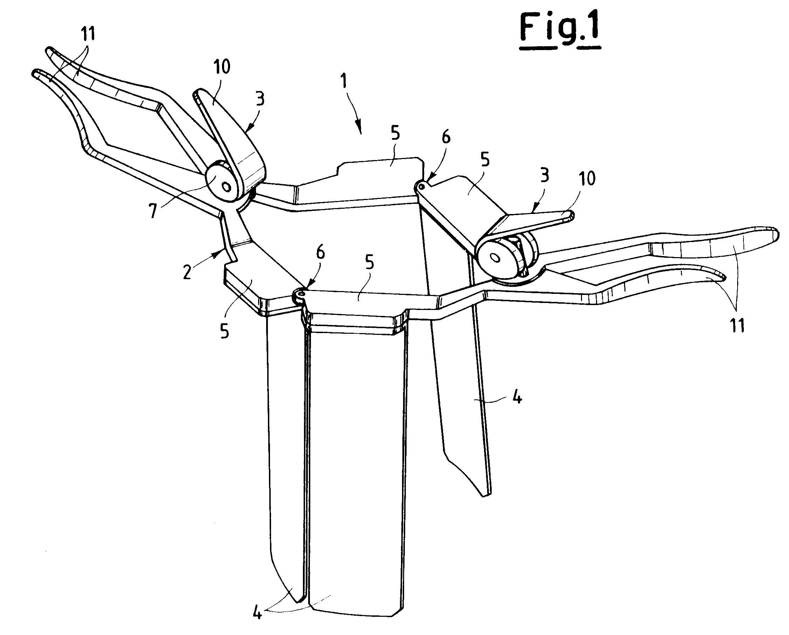

On approaching the arms 11 the retractor frame 2 opens to assume the configuration shown in Figure 1. When in the final required configuration, which is achieved by approaching the arms 11 to a greater or lesser degree (and hence opening the edges of the incision to a greater or lesser extent), the retractor 1 is locked by the means 3. This is done by pressing on the levers 10, by which the cams 7 act on the pins 8A to rigidly lock the engagement of the pairs of toothed jaws 9.

Because of the convergence of the axes 16A of the hinges 6A, the retractor frame 2 in passing from the closed to the open configuration changes the plane in which it lies. Consequently, in addition to withdrawing from each other, the dilators 4 incline so that the bevelled ends 13 project outwards from the retractor 1 to enlarge the effective operating area beyond the extent by which the edges of the incision are withdrawn from each other, and hence more than the width of the incision itself.

It is apparent that in this manner a relatively large effective operating area within the patient's body is available with relatively small incisions.

The retractor frame 2 and dilators 4 can be of metal or plastic material, possibly provided in packs for once-only use.

In an alternative embodiment the dilators 4 can be constructed of materials able to emit light when traversed by light rays.

With particular reference to Figure 5, the dilators 4 can each be provided with a supplementary dilator 14 hinged at 15 to the dilator 4 such that when the supplementary dilator 14 is superposed on the dilator 4 they have one of their edges parallel to each other and coincident. In the illustrated example, the supplementary dilator 14 is triangular and has a smaller area than the dilator 4. In other embodiments the supplementary dilator 14 can be of such an extension as to reach the lower end of the dilator 4 to which it is hinged.

The supplementary dilators 14, which can rotate about the hinge 15, are made to project laterally from the respective supporting dilators 4 after the retractor 1 has been inserted into the patient's body, so helping the dilators 4 to which they are hinged to better retain the organs not concerned in the operation, thus better defining the effective area concerned in the operation.

## Claims

1. A surgical retractor (1) particularly for cholecystectomy, comprising:
- a retractor frame (2) formed by a series of sides (5) connected to each other by hinges (6, 6A) to form an articulated polygonal frame;
- means for locking said retractor frame (2) in an autostatic position operating on a pair of opposite hinges (6A);
- a plurality of dilators (4) secured to the sides (5) of the retractor frame (2), said dilators (4) being of substantially thin plate form and extending along an axis which cuts the retractor frame (2),
characterised in that the axes (16A) of a pair of opposite hinges (6A) converge at a point situated opposite to the dilators (4) relative to the retractor frame (2), and in that at said hinges (6A) with converging axes the hinged sides (5) are prolonged to form a pair of arms (11) approachable to each other to widen and hence open the retractor frame (2).

2. A surgical retractor as claimed in claim 1, characterised in that the converging axes (16A) form an angle between 50° and 60°.

3. A surgical retractor as claimed in claim 1, characterised in that the means for locking said retractor frame (2) in an autostatic position are arranged at said opposite hinges (6A) having the converging axes.

4. A surgical retractor as claimed in claim 1 or 3, characterised in that said locking means comprise two cams (7) manually operable by respective levers (10) and acting via respective pins (8A) of the hinges (6A) on respective pairs of toothed jaws (9) rigid with two mutually hinged sides (5) of the retractor frame (2).

5. A surgical retractor as claimed in claim 1, characterised in that the number of dilators (4) is at least one less than the number of sides (5) of the retractor frame (2).

6. A surgical retractor as claimed in claim 1 or 5, characterised in that the dilators (4) are removably secured to the sides (5) of the retractor frame (2).

7. A surgical retractor as claimed in one of claims 1, 5 or 6, characterised in that the dilators (4) have a substantially arcuate cross-section with its concavity facing the interior of the retractor (1).

8. A surgical retractor as claimed in one of claims 1, 5, 6 or 7, characterised in that the dilators (4) are constructed of a material capable of emitting light when traversed by light rays.

9. A surgical retractor as claimed in one of claims 1, 5, 6, 7 or 8, characterised in that supplementary dilators (14) are hingedly supported by said dilators (4).

10. A surgical retractor as claimed in claim 9, characterised in that the supplementary dilators (14) are of triangular shape and are hinged at a vertex so that when the hinged dilators (4, 14) are superposed one edge of the supplementary dilators (14) is parallel to one edge of the supporting dilators (4).

## Patentansprüche

1. Chirurgischer Retraktor (1), insbesondere zur Cholezystektomie, enthaltend:
- einen Retraktorrahmen (2), der von einer Reihe von Seitenteilen (5) gebildet ist, die miteinander durch Gelenke (6, 6A) verbunden sind, so daß ein gelenkiger polygonaler Rahmen gebildet wird;
- eine Einrichtung zur Fixierung dieses Retraktorrahmens (2) in einer autostatischen Stellung, welche Einrichtung auf ein Paar einander gegenüberliegender Gelenke (6A) wirkt;
- eine Vielzahl von an den Seitenteilen (5) des Retraktorrahmes (2) befestigten Dilatoren (4), welche im wesentlichen die Gestalt dünner Platten haben und sich entlang einer Achse erstrecken, welche den Retraktorrahmen (2) schneidet,
dadurch gekennzeichnet, daß die Achsen (16A) eines Paares einander gegenüberliegender Gelenke (6A) gegen einen Punkt konvergieren, der auf der den Dilatoren (4) abgewendeten Seite des Retraktorrahmens (2) liegt, und daß an diesen Gelenken (6A) mit konvergierenden Achsen die angelenkten Seitenteile (5) zur Bildung eines Paares von Armen (11) verlängert sind, welche einander zur Aufweitung und damit zur Öffnung des Retraktorrahmens (2) genähert werden können.

2. Chirurgischer Retraktor nach Anspruch 1, dadurch gekennzeichnet, daß die konvergierenden Achsen (16A) einen Winkel zwischen 50° und 60° einschließen.

3. Chirurgischer Retraktor nach Anspruch 1, dadurch gekennzeichnet, daß die Einrichtung zur Fixierung des Retraktorrahmes (2) in einer autostatischen Stellung an den einander gegenüberliegenden Gelenken (6A) mit konvergierenden Achsen angeordnet ist.

4. Chirurgischer Retraktor nach Anspruch 1 oder 3, dadurch gekennzeichnet, daß die Fixiereinrichtung zwei Nocken (7) aufweist, die jeweils durch Hebel (10) händisch betätigtbar sind und über zugeordnete Zapfen (8A) der Gelenke (6A) auf zugeordnete Paare gezahnter Backen (9) wirken, die fest mit zwei miteinander gelenkig verbundenen Seitenteilen (5) des Retraktorrahmens (2) verbunden sind.

5. Chirurgischer Retraktor nach Anspruch 1, dadurch gekennzeichnet, daß die Anzahl der Dilatoren (4) zumindest um eins geringer ist als die Anzahl der Seitenteile (5) des Retraktorrahmens (2).

6. Chirurgischer Retraktor nach Anspruch 1 oder 5, dadurch gekennzeichnet, daß die Dilatoren (4) abnehmbar an den Seitenteilen (5) des Retraktorrahmens (2) befestigt sind.

7. Chirurgischer Retraktor nach einem der Ansprüche 1, 5 oder 6, dadurch gekennzeichnet, daß die Dilatoren (4) einen im wesentlichen gekrümmten Querschnitt aufweisen, dessen konkave Seite dem Inneren des Retraktors (1) zugewendet ist.

8. Chirurgischer Retraktor nach einem der Ansprüche 1, 5, 6 oder 7, dadurch gekennzeichnet, daß die Dilatoren (4) aus einem Material ausgebildet sind, das bei Beaufschlagung durch Lichtstrahlen Licht aussendet.

9. Chirurgischer Retraktor nach einem der Ansprüche 1, 5, 6, 7 oder 8, dadurch gekennzeichnet, daß zusätzliche Dilatoren (14) von den Dilatoren (4) gelenkig getragen sind.

10. Chirurgischer Retraktor nach Anspruch 9, dadurch gekennzeichnet, daß die zusätzlichen Dilatoren (14) dreieckige Gestalt aufweisen und an einem Eckpunkt derart angelenkt sind, daß im übereinandergelegten Zustand der gelenkig verbundenen Dilatoren (4,14) eine Kante des zusätzlichen Dilators (14) parallel zu einer Kante des tragenden Dilators (4) verläuft.

## Revendications

1. Ecarteur chirurgical (1), particulièrement destiné à la cholécystectomie, comprenant:
- un cadre écarteur (2) formé par une série de côtés (5) reliés les uns aux autres par des articulations (6, 6A) pour former un cadre polygonal articulé;
- des moyens de verrouillage dudit cadre écarteur (2) dans une position autostatique agissant sur une paire d'articulations opposées (6A);
- une pluralité de dilatateurs (4) fixés aux côtés (5) sur le cadre écarteur (2), lesdits dilatateurs (4) étant d'une forme en plaque substantiellement mince et s'étendant le long d'un axe qui coupe le cadre écarteur (2),
caractérisé en ce que les axes (16A) d'une paire d'articulations opposées (6A) convergent en un point situé à l'opposé des dilatateurs (4) par rapport au cadre écarteur (2), et en ce que, sur lesdites articulations (6A) à axes convergeants, les côtés articulés (5) sont prolongés pour former une paire de bras (11) pouvant être approchés l'un de l'autre pour élargir et donc ouvrir le cadre écarteur (2).

2. Ecarteur chirurgical selon la revendication 1, caractérisé en ce que les axes convergeants (16A) forment un angle entre 50° et 60°.

3. Ecarteur chirurgical selon la revendication 1, caractérisé en ce que les moyens de verrouillage dudit cadre écarteur (2) dans une position autostatique sont disposés sur lesdites articulations opposées (6A) ayant des axes convergeants.

4. Ecarteur chirurgical selon la revendication 1 ou 3, caractérisé en ce que lesdits moyens de verrouillage comprennent deux cames (7) actionnables manuellement par des leviers respectifs (10) et agissant par l'intermédiaire de broches respectives (8A) des articulations (6A) sur des paires respectives de mâchoires dentées (9) rigides avec deux côtés mutuellement opposés (5) du cadre écarteur (2).

5. Ecarteur chirurgical selon la revendication 1, caractérisé en ce que le nombre de dilatateurs (4) est au moins un de moins que le nombre de côtés (5) du cadre écarteur (2).

6. Ecarteur chirurgical selon la revendication 1 ou 5, caractérisé en ce que les dilatateurs (4) sont fixés de manière amovible aux côtés (5) du cadre écarteur (2).

7. Ecarteur chirurgical selon l'une des revendications 1, 5 ou 6, caractérisé en ce que les dilatateurs (4) ont une section transversale substantiellement arquée avec sa concavité tournée vers l'intérieur de l'écarteur (1).

8. Ecarteur chirurgical selon l'une des revendications 1, 5, 6 ou 7, caractérisé en ce que les dilatateurs (4) sont construits dans un matériau capable d'émettre de la lumière lorsqu'il est traversé par des rayons lumineux.

9. Ecarteur chirurgical selon l'une des revendications 1, 5, 6, 7 ou 8, caractérisé en ce que des dilatateurs supplémentaires (14) sont portés de manière articulée par lesdits dilatateurs (4).

10. Ecarteur chirurgical selon la revendication 9, caractérisé en ce que les dilatateurs supplémentaires (14) sont de forme triangulaire et sont articulés en un sommet de sorte que, lorsque les dilatateurs articulés (4, 14) sont superposés, un bord des dilatateurs supplémentaires (14) est parallèle à un bord des dilatateurs porteurs (4).
